# EUROPÄISCHE PATENTSCHRIFT

(11) **EP 1 007 022 B1**
(45) Veröffentlichungstag und Bekanntmachung des Hinweises auf die Patenterteilung: **11.07.2001**
(21) Anmeldenummer: 98908081.7
(22) Anmeldetag: 11.02.1998
(51) Int. Cl.: A61K 31/12, A61K 35/42

(54) **ZUBEREITUNG DES PULMONARY SURFACTANT ZUR INSTILLATION UND ZUR ORALEN ANWENDUNG**
PREPARATION OF A PULMONARY SURFACTANT FOR INSTILLATION AND ORAL APPLICATION
PREPARATION D'Un SURFACTANT PULMONAIRE A DES FINS D'INSTILLATION ET D'ADMINISTRATION ORALE

(30) Priorität: 12.02.1997 DE 19705230
(43) Veröffentlichungstag der Anmeldung: 14.06.2000
(73) Patentinhaber: MSE Pharmazeutika GmbH, 61348 Bad Homburg (DE)
(72) Erfinder: ENZMANN, Franz, D-61348 Bad Homburg (DE); LACHMANN, Burkhard, NL-3065 BC Rotterdam (NL)
(74) Vertreter: Werner, Hans-Karsten, Dr.Dipl.-Chem.
(86) Internationale Anmeldenummer: EP9800745
(87) Internationale Veröffentlichungsnummer: WO9835661

(56) Entgegenhaltungen:
- DATABASE WPI Section Ch, Week 9704 Derwent Publications Ltd., London, GB; Class B04, AN 97-036508 XP002069687 & ES 2 092 969 A (CENSA CENT NACIONAL SANIDAD AGROPECUARIA)

## Beschreibung

Der Pulmonary Surfactant ist ein Komplex von Phospholipiden, neutralen Lipiden und Surfactant Proteinen, die miteinander eine einschichtige Barriere zwischen der Luft und der flüssigen Oberfläche der Lunge ausbilden. Der Pulmonary Surfactant wird in den Alveolaren Typ II Zellen gebildet und von dort in der Alveolar-Zwischenraum abgegeben. Der Pulmonary Surfactant kann extraktiv und rekombiniert gewonnen werden.

Der Pulmonary Surfactant ist bisher eingesetzt worden zur Instillation bei Erkrankungen und Mangelerscheinungen der Lunge; insbesondere beim IRDS, dem Atemnot-Syndrom bei Kinder und beim ARDS.

Es wurde jetzt gefunden auch zur oralen Behandlung von Erkrankungen des gesamten Verdauungstraktes (Mund, Trachea, Magen und Darm), daß sich die Wirksamkeit und die Haltbarkeit des Pulmonary Surfactants steigern läßt durch einen Zusatz von mindestens 0,1 Gew.-% 2,3-Dimethoxy-5-methyl-6-decaprenyl-1,4-ben-zochinon.

2,3-Dimethoxy-5-methyl-6-decaprenyl-1,4 -benzochinon ist auch bekannt unter der Bezeichnung Coenzym Q10. Die Substanz spielt eine Rolle in der Atmungskette und ist obendrein ein Antioxidanz, welches in der Lage ist, Radikale, die insbesondere von Vitaminen weitergegeben werden, abzufangen und unschädlich zu machen. Q-10 bestimmt außerdem die Elastizität und die Dynamik der Zellmembranen. Es wird daher bisher als Monopräparat und in Kombination mit anderen Wirkstoffen zur oralen Einnahme empfohlen. Weiterhin wird es zur Hautpflege in Form einer Liposomencreme angeboten, welche es dem Wirkstoff gestattet, durch die Hornschichtbarrieren einzudringen und sich dann in den verschiedenen Schichten der Haut anzureichern. Aus diesem ergibt sich jedoch kein Hinweis darauf, daß bereits kleine Mengen dieser Substanz in der Lage sind, den Wirkstoff Pulmonary Surfactant zu stabilisieren und seine Wirksamkeit zu steigern. Insbesondere höhere Mengen des Wirkstoffes 2,3-Dimethoxy-5-methyl-6-decaprenyl-1,4-benzochinon sind in der Lage, bei Instillation in die Lunge das lungen-ständige Immunsystem zu stärken, der Zelle zu helfen, geschädigte Zellstrukturen wieder aufzubauen, und dabei gleichzeitig das Pulmonary Surfactant hinsichtlich seiner Wirksamkeit zu steigern.

Überraschend ist, daß Q-10 nicht nur den Lipiddoppelmembranen, sondern auch dem Pulmonary Surfactant-System Dynamik verleiht.

Gegenstand der vorliegenden Erfindung sind somit Zubereitungen des Pulmonary Surfactant zur Instillation enthaltend außer einer wirksamen Menge des Pulmonary Surfactant und üblichen Hilfsstoffen mindestens 0,1 Gew.-% 2,3-Dimethoxy-5-methyl-6-decaprenyl-1,4-benzochinon.

Die Menge an 2,3-Dimethoxy-5-methyl-6-decaprenyl-1,4-benzochinon kann bis zum Gewichtsverhältnis 1:1 mit dem Pulmonary Surfactant erhöht werden, wodurch besondere therapeutische Effekte erzielt werden.

## Patentansprüche

1. Zubereitung des Pulmonary Surfactant zur Instillation und oralen Anwendung enthaltend außer einer wirksamen Menge des Pulmonary Surfactant und üblichen Hilfsstoffen mindestens 0,1 Gew.-% 2,3-Dimethoxy-5-methyl-6-decaprenyl-1,4-benzochinon.

## Claims

1. Preparation of the pulmonary surfactant for the instillation and oral application containing in addition to an effective amount of said pulmonary surfactant and conventional auxiliary agents at least 0.1 % by weight of 2,3-dimethoxy-5-methyl-6-decaprenyl-1,4-benzoquinone.

## Revendications

1. Préparation du surfactant pulmonaire pour l'instillation et l'application orale contenant en plus d'une quantité efficace du surfactant pulmonaire et des agents auxiliaires au moins 0,1 % en poids de 2,3-diméthoxy-5-méthyl-6-décaprényl-1,4-benzoquinone.
